# EUROPEAN PATENT SPECIFICATION

(11) **EP 2 376 070 B1**
(45) Date of publication and mention of the grant of the patent: **10.08.2016**
(21) Application number: 09799316.6
(22) Date of filing: 14.12.2009
(51) Int. Cl.: A61K 9/00, A61K 9/16, A61K 38/31

(54) **OCTREOTIDE DEPOT FORMULATION WITH CONSTANTLY HIGH RELEASE RATES**
OCTREOTID-DEPOTFORMULIERUNG MIT KONSTANT HOHE FREISETZUNGSRATE
FORMULATION À EFFET RETARD D'OCTRÉOTIDE AVEC UN HAUTE TAUX DE LIBÉRATION CONSTANT

(30) Priority: 15.12.2008 EP 08171712
(43) Date of publication of application: 19.10.2011
(73) Proprietor: Novartis AG, 4056 Basel (CH)
(72) Inventor: AHLHEIM, Markus, CH-4002 Basel (CH); PETERSEN, Holger, CH-4002 Basel (CH)
(74) Representative: Pfister-Fu, Yixin
(86) International application number: PCT/EP2009/067049
(87) International publication number: WO 2010/079047

(56) References cited:
- WO-A1-2005/046645
- WO-A2-2004/043432
- PAUL J. FLORY: 'Principles of Polymer Chemistry', CORNELL UNIVERSITY PRESS * page 308 *

## Description

The present invention relates to sustained release formulations comprising as active ingredient octreotide or a pharmaceutically-acceptable salt thereof and certain linear polylactide-co-glycolide polymers (PLGAs).

The pharmaceutical compositions according to the present invention are indicated for inter alia long-term maintenance therapy in acromegalic patients, and treatment of severe diarrhea and flushing associated with malignant carcinoid tumors and vasoactive intestinal peptide tumors (vipoma tumors).

Peptide drugs are usually administered systemically, e.g. parenterally. However, parenteral administration may be painful and cause discomfort, especially for repeated daily administrations. In order to minimize the number of injections to a patient, the drug substance is advantageously administered as a depot formulation. A common drawback with injectable depot formulations is the fluctuation in plasma levels such as high peak levels together with plasma levels close to zero during the entire release period.

The present invention now provides an improved depot formulation providing constantly high exposure level. Furthermore, the depot formulation of the present invention reach the exposure level rapidly, i.e. have only a short or no lag phase. The depot formulations of the present invention comprise as active ingredient (drug substance) octreotide or a pharmaceutically-acceptable salt thereof. Octreotide is a somatostatin analog having the following formula:

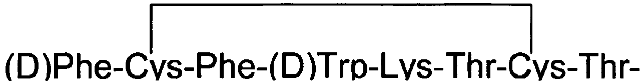

The active ingredient may be in the form of a pharmaceutically acceptable salt of octreotide, such as an acid addition salt with e.g. inorganic acid, polymeric acid or organic acid, for example with hydrochloric acid, acetic acid, lactic acid, citric acid, fumaric acid, malonic acid, maleic acid, tartaric acid, aspartic acid, benzoic acid, succinic acid or pamoic (embonic) acid. Acid addition salts may exist as mono- or divalent salts, e.g. depending whether 1 or 2 acid equivalents are added. Preferred is the pamoate monosalt of octreotide.

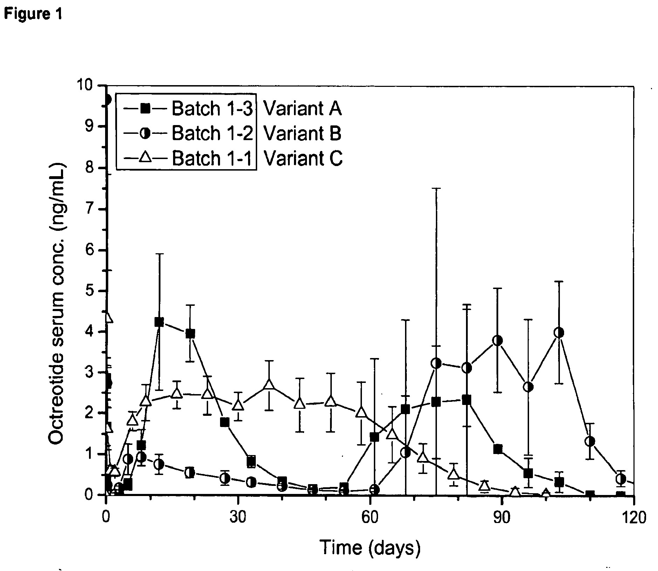

To sufficiently control the hGH and IGF-1 levels of acromegaly patients a constant octreotide plasma level of as high as at least 1.5 ng/ml, 1.8 ng/ml or 2 ng/ml are required to sufficiently control the disease (therapeutic target plasma concentration). Developing a PLGA depot formulation which can constantly achieve these high plasma levels over an extended period of time has been proven very challenging. So far, none of the described octreotide depot formulations are able to meet the target plasma level with a dosage of 12 mg/kg body weight in rabbits (Male New Zealand White rabbits (Hsdlf:NZW), ~ 3 kg ± 20% at arrival (Harlan Netherlands)) over an extended time of more than 50 days. Sustained release formulations comprising as active ingredient octreotide or a pharmaceutically acceptable salt thereof and polylactide-co-glycolide polymers (PLGAs) have been described, for instance, in GB2265311 or WO2007/071395. However, the prior art formulations show either long phases of low levels ("lag phases") as Batch 1-2 described in Figure 1 and/or in between of the diffusion controlled release and the erosion controlled release a "valley" as Batch 1-2 and 1-3 described in Figure 1. Additional relevant prior art includes WO2005 04 6645.

It has now surprisingly found in accordance with the present invention that formulations comprising two different linear PLGA polymers having a lactide:glycolide comonomer (L:G) ratio of 75:25 and different viscosities provide a favorable release profile, in particular with respect to lag phase or the valley. The formulations of the present invention have been found to be able to provide sustained high octreotide plasma levels of at least 1.5 ng/ml, 1.8 ng/ml or 2 ng/mL for extended period of time such as e.g. at least 50 days. The favorable release profile over an extended time is therefore particularly suitable for a sustained release formulation which can be applied over a longer time than currently marketed sustained release formulation of octreotide, also know as Sandostatin® LAR®, which is administered every 28 days.

In one aspect, the present invention provides an octreotide depot formulation composed of a blend of two different PLGA polymers both of a L:G ratio of 75:25 but of different inherent viscosities. The different polymers have different end groups an ester and a carboxy end group. The formulation shows constantly a high exposure for at least 50 days, preferably at least about 2 months, in rabbits after i.m. injection. Furthermore, the depot formulations of the present invention show a short lag phase until the therapeutic target level is reached. For a single injection, a typical lag phase between the initial burst and reaching the therapeutic target plasma concentration of a formulation of the present invention is shorter than 12 days, e.g. between 4 to 12 days or 6 to 10 days.

The particle size distribution of the drug substance influences the release profile of the drug from the depot form. The drug substance which is used to prepare the depot formulation is crystalline or in the form of an amorphous powder. Preferred is an amorphous powder which has a particle of a size of about 0.1 microns to about 15 microns (99% > 0.1 microns, 99% < 15 microns), preferably from 1 to less than about 10 microns (90% > 1 microns, 90% < 10 microns). The drug substance preferentially undergoes a micronization process to present the required particle size distribution.

The present invention further provides a sustained release pharmaceutical composition (depot) comprising as active ingredient octreotide or a pharmaceutically-acceptable salt thereof incorporated in a poly(lactide-co-glycolide)s (PLGAs) matrix, for instance in form of microparticles, implants or semisolid formulations.

The pharmaceutical composition according to the present invention allows a sustained release of the active ingredient in a patient in need (preferably a human) over a period of at least 45 days, at least 50 days, at least 60 days, at least 75 days or at least 90 days. The pharmaceutical composition of the present invention allows a sustained release of the active ingredient between 60 to 120 days. During the release of the active ingredient the plasma levels of octreotide are within the therapeutic range. It is understood that the exact dose of octreotide will depend on a number of factors, including the condition to be treated, the severity of the condition to be treated, the weight of the subject and the duration of therapy. The favorable release profile of the present invention allows for longer administration intervals of the pharmaceutical compositions of the present invention as compared to the prior art formulations. So far no octreotide depot formulation with longer dosing intervals than every 28 days have been approved for therapy. The depot formulations of the present invention are now, due to their favorable release profile, suitable for administration once every 2 months (e.g. every 8 weeks or every 60 days) up to once every 4 months (e.g. every 16 weeks or every 120 days). In one preferred embodiment, the depot formulation of the present invention are administered once every 3 months (e.g. every 12 weeks or every 90 days).

Surprisingly fluctuations in plasma levels can are significantly reduced by using a suitable combination of two different linear PLGAs in the pharmaceutical composition according to the present invention.

The drug substance is incorporated into a biodegradable polymer matrix consisting of two different linear polylactide-co-glycolide polymers (PLGAs). The PLGAs have a lactide: glycolide monomer ratio of 75:25.

The PLGAs according to the present invention have a molecular weight (Mw) ranging from 1,000 to 500,000 Da, preferably from 5,000 to 100,000 Da. The architecture of the polymers is linear.

The inherent viscosity (IV) of the PLGAs according to the present invention is below 0.9 dl/g in CHCl₃, preferentially below 0.8 dl/g, preferably below 0.6 dl/g, more preferably between 0.1 dl/g to 0.5 dl/g in CHCl₃. The inherent viscosities can be measured by the conventional methods of flow time measurement, as described for example in "Pharmacopoée Européenne", 1997, pages 17-18 (capillary tube method). Unless stated otherwise, these viscosities have been measured at 25°C at a concentration of 0.1 % in CHCl₃.

End groups of the PLGAs according to the present invention can be but are not limited to Hydroxy, carboxy, ester.

The drug substance content of the depot formulation (the loading) is in a range of 1% to 30%, preferred 10% to 25%, more preferred 15% to 20%. The loading is defined as the weight ratio of drug substance as free base to the total mass of the PLGA formulation.

Suitable polymers are commonly known but not limited to those commercially available as RESOMER® by Boehringer Ingelheim Pharma GmbH & Co. KG, Ingelheim, Germany, LACTEL® by Durect Corp., Pelham, AL, USA, MEDISORB® by Lakeshore, Inc., Cambridge, MA, USA, PURASORB® by PURAC biochem BV, Gorinchem, The Netherlands. Particularly preferred polymers of the present invention are Resomer® RG 752 H and Resomer ® RG 753 S.

The pharmaceutical composition according to the present invention can be manufactured aseptically or non-aseptically and sterilized terminally by gamma irradiation. Preferred is terminal sterilization by gamma irradiation, resulting in a product with the highest sterility assurance possible.

The pharmaceutical composition according to the present invention may also contain one or more pharmaceutical excipients modulating the release behavior in an amount of 0.1% to 50%. Examples of such agents are: Polyvinyl alcohol, Polyvinyl pyrrolidone, carboxymethyl cellulose sodium (CMC-Na), dextrin, polyethylene glycol, suitable surfactants such as poloxamers, also known as poly(oxyethylene-block-oxypropylene), Poly(oxyethylene)-sorbitan-fatty acid esters known and commercially available under the trade name TWEEN® (e.g. Tween 20, Tween 40, Tween 60, Tween 80, Tween 65 Tween 85, Tween 21, Tween 61, Tween 81), Sorbitan fatty acid esters e.g. of the type known and commercially available under the trade name SPAN, Lecithins, inorganic salts such as zinc carbonate, magnesium hydroxide, magnesium carbonate, or protamine, e.g. human protamine or salmon protamine, or natural or synthetic polymers bearing amine-residues such as polylysine .

The pharmaceutical composition according to the present invention can be a depot mixture or a polymer blend of different polymers in terms of compositions, molecular weight and/or polymer architectures. A polymer blend is defined herein as a solid solution or suspension of two different linear polymers in one implant or microparticle. A mixture of depots in contrast is defined herein as a mixture of two depots like implants or microparticles or semisolid formulations of different composition with one or more PLGAs in each depot. Preferred is a pharmaceutical composition wherein the two PLGAs are present as polymer blend.

The pharmaceutical composition according to the present invention can be in the form of implants, semisolids (gels), liquid solutions or suspensions which solidify in situ once they are injected or microparticles. Preferred are microparticles. Preparation of microparticles comprising octreotide or a pharmaceutically-acceptable salt thereof is known and for instance disclosed in US5,445,832 or US5,538,739.

The following part of the invention is focused on polymer microparticles although the descriptions are applicable for implants, semisolids and liquids as well.

The microparticles according to the present invention may have a diameter from a few submicrons to a few millimeters, e.g. from 0.01 microns to 2 mm, e.g. from 0.1 microns to 500 microns. For pharmaceutical microparticles, diameters of at most 250 microns, e.g. 10 to 200 microns, preferably 10 to 130 microns, more preferably 10 to 90 microns.

The microparticles according to the present invention may be mixed or coated with an anti-agglomerating agent or covered by a layer of an anti-agglomerating agent, e.g. in a prefilled syringe or vial. Suitable anti-agglomerating agents include, e.g. mannitol, glucose, dextrose, sucrose, sodium chloride, or water soluble polymers such as polyvinyl alcohol, polyvinyl pyrrolidone or polyethylene glycol, e.g. with the properties described above.

The manufacturing process for the depot formulation of the current invention is described in detail for microparticles:
The microparticles may be manufactured by several processes known in the art, e.g., coacervation or phase separation, spray drying, water-in-oil (W/O) or water-in-oil-in-water (W/O/W) or solids-in-oil-in-water (S/O/W) emulsion/suspension methods followed by solvent extraction or solvent evaporation. The emulsion/suspension method is a preferred process, which comprises the following steps:
   (i) preparation of an internal organic phase comprising
      (ia) dissolving the polymer or polymers in a suitable organic solvent or solvent mixture; optionally dissolving/dispersing suitable additives;
      (ib) dissolving/suspending/emulsification of the drug substance in the polymer solution obtained in step (ia);
   (ii) preparation of an external aqueous phase containing stabilizers and optionally but preferably buffer salts;
   (iii) mixing the internal organic phase with the external aqueous phase e.g. with a device creating high shear forces, e.g. with a rotor-stator mixer (turbine) or static mixer, to form an emulsion; and
   (iv) hardening the microparticles by solvent evaporation or solvent extraction, washing the microparticles, e.g. with water, collecting and drying the microparticles, e.g. freeze-drying or drying under vacuum, and sieving the microparticles through 140 µm.

Suitable organic solvents for the polymers include e.g. ethyl acetate, acetone, THF, acetonitrile, or halogenated hydrocarbons, e.g. methylene chloride, chloroform or hexafluoroisopropanol.

Suitable examples of a stabilizer for step (iib) include Poly(vinylalcohol) (PVA), in an amount of 0.1 to 5%, Hydroxyethyl cellulose (HEC) and/or hydroxypropyl cellulose (HPC), in a total amount of 0.01 to 5%, Poly(vinyl pyrolidone), Gelatin, preferably porcine or fish gelatin.

The dry microparticles composition can be terminally sterilized by gamma irradiation (overkill sterilization), optionally in bulk or after filling in the final container resulting in the highest sterility assurance possible. Alternatively the bulk sterilized microparticles can be resuspended in a suitable vehicle and filled as a suspension into a suitable device such as double chamber syringe with subsequent freeze drying.

The pharmaceutical composition according to the present invention containing microparticles may also contain a vehicle to facilitate reconstitution.

Prior to administration, the microparticles are suspended in a suitable vehicle for injection. Preferably, said vehicle is water based containing pharmaceutical excipients such as mannitol, sodium chloride, glucose, dextrose, sucrose, or glycerins, non-ionic surfactants (e.g. poloxamers, poly(oxyethylene)-sorbitan-fatty acid esters, carboxymethyl cellulose sodium (CMC-Na), sorbitol, poly(vinylpyrrolidone), or aluminium monostearate in order to ensure isotonicity and to improve the wettability and sedimentation properties of the microparticles. The wetting and viscosity enhancing agents may be present in an amount of 0.01 to 1%; the isotonicity agents are added in a suitable amount to ensure an isotonic injectable suspension.

The invention further provides the use of a pharmaceutical composition according to the present invention for inter alia long-term maintenance therapy in acromegalic patients, and treatment of severe diarrhea and flushing associated with malignant carcinoid tumors and vasoactive intestinal peptide tumors (vipoma tumors).

The utility of the pharmaceutical compositions according to the present invention can be shown in standard clinical or animal studies.

The invention further provides a kit comprising the depot formulation in a vial, optionally equipped with a transfer set, together with a water-based vehicle in an ampoule, vial or prefilled syringe or as microparticles and vehicle separated in a double chamber syringe.

### Brief Description of the Drawings

FIG. 1 shows examples 1-1, 1-2 and 1-3 (formulation variants C, B, A and in comparison. Octreotide serum conc. over time after 12 mg/kg dosage i.m. into rabbits. Mean and SD of 4 animals.

### Experimental Part

### Example 1: Microparticle preparation

An appropriate amount of the PLGA polymers is dissolved in an appropriate amount of dichloromethane to give an appropriate polymer concentration as stated in column "PLGA conc." in Table 2. An appropriate amount of drug substance is weight into a glass beaker and the polymer solution is poured over the drug substance so that the resulting microparticles have a drug load as stated in column "drug load".

E.g. for microparticles with a drug load of 20% and a polymer concentration of 20% the numbers are as the following: 3.547 g of the PLGA polymers are dissolved into 17.7 ml dichloromethane to give a 20 % (w/v) polymer solution. 1.453 g of octreotide pamoate with a free peptide content of 68.8% (corresponding to 1.00 g = 20% octreotide free base) is weight into a glass beaker and the polymer solution is poured over the drug substance.

The suspension is homogenized with an Ultra-Turrax rotor-stator mixer with 20'000 rpm for 1 min under cooling with an ice/water mixture. This suspension is referred to as S/O suspension.

10.00 g of Polyvinylalcohol PVA 18-88, 3.62 g KH₂PO₄ and 15.14 g Na₂HPO₄ are dissolved in 2.00 L deionized water to form a 0.5% PVA 18-88 solution buffered to pH 7.4.

The S/O suspension is mixed with the 0.5 % PVA18-88 solution by pumping the S/O suspension with the help of a flexible tube pump (Perpex, Viton tube) at a rate of 10 ml/min into a turbine and by pumping the aqueous solution with a gear pump (Ismatec MV-Z/B with pumping head P140) at a rate of 200 ml/min into the same turbine. The two solutions are mixed in the turbine as described in Table 2. The homogenized S/O/W emulsion is collected into a 2 L glass beaker which is prefilled with 200 ml of the buffered PVA solution.

The S/O/W emulsion is then heated up to 45°C in 5 h. The temperature of 45°C is hold for further 2 h min, before the batch is cooled to room temperature again. During this process escaping dichloromethane is removed by vacuum and the batch is stirred by a 4 blade-propeller-stirrer at 250 rpm.

As a result, microparticles are formed out of the S/O/W emulsion. The microparticles are collected by filtration (5 µm). They are washed 5 times with 200 ml water and dried for 36 h at 20°C and 0.030 mbar. The dried microparticles are sieved through 140 µm and filled under nitrogen into glass vials. Prepared in that way, the microparticles are sterilized by gamma-irradiation with a dose of 30 kGy.

The particle size of the microparticles is measured by laser light diffraction. The microparticles are resuspended in white spirit using ultra sound. Table 2 gives the diameter x90 (90% of all particles are smaller than this value) after 120 seconds of ultra sound treatment.

The assay of the microparticles is determined by HPLC after dissolving the microparticles with ultra sound in a 3:2 mixture of acetonitrile and methanol and further 1:1 dilution with a sodium acetate buffer (pH 4). The solution is cleared from residual particulate matter by centrifugation.

**Table 2: Examples 1-1: octreotide pamoate microparticles prepared by blend of two linear PLGAs (75:25). Comparative examples 1-2 and 1-3: octreotide pamoate microparticles prepared by blend of two or three linear PLGAs.**

| **Ex. Batch** | **Drug Load (%)** | **PLGA conc. (%)** | **A** | **B** | **C** | **D** | **Turbine speed (rpm)** | **Particle size x₉₀ (µm)** | **Assay (%)** |
|---|---|---|---|---|---|---|---|---|---|
| **1-1 VarC** | 20 | 20 | | 30 | 70 | -- | 2800 | 60 | 18.4 |
| **1-2 VarB** | 20 | 20 | 33 | -- | 34 | 33 | 3800 | 68.4 | 19.6 |
| **1-3 Var A** | 20 | 20 | -- | -- | 50 | 50 | 4500 | 58.6 | 18.6 |

| | | | | | | | | | |
|---|---|---|---|---|---|---|---|---|---|
| **A:** PLGA 65:35 ester 0.6 dL/g (%) **B:** PLGA 75:25 acid 0.2 dL/g (%) **C:** PLGA 75:25 ester 0.4 dL/g (%) **D:** PLGA 85:15 ester 0.6 dL/g (%) | | | | | | | | | |

### Examples 2: Vehicle compositions A to G

CMC-Na, Mannitol and Pluronic F68 in an amount as given in Table 3 are dissolved in about 15 ml hot deionized water of a temperature of about 90°C under strong stirring with a magnetic stirrer. The resulting clear solution is cooled to 20°C and filled up with deionized water to 20.0 ml.

**Table 3: Suitable vehicles for the microparticles (Amounts given in g)**

| | **A** | **B** | **C** | **D** | **E** | **F** | **G** |
|---|---|---|---|---|---|---|---|
| CMC-Na | 0 | 0 | 0.05 | 0.14 | 0.28 | 0.35 | 0.40 |
| Mannitol | 0 | 1.04 | 0.99 | 0.90 | 0.76 | 0.74 | 0.68 |
| Pluronic F68 | 0.04 | 0.04 | 0.04 | 0.04 | 0.04 | 0.04 | 0.04 |

### Example 3: Microparticle suspension

180 mg of microparticles of example 1-1, 1-2 or 1-3 are suspended in 1.0 ml of a vehicle of composition D (Table 3) in a 6 R vials. The suspensions are homogenized by shaking for about 30 seconds by hand. The reconstituted suspension may be injected without any issues using a 20 Gauge needle.

### Example 4: Lyophilisation of the microparticles

180 mg of microparticles of example 1-1, 1-2 or 1-3 are reconstituted in 1 ml of the vehicle composition F (Table 3), homogenized by stirring for 1 to 12 hours and then freeze-dried in a lyophilisator. Reconstitution of the lyophilized microparticles with 1 ml pure water (aqua ad injectabilia) resulted in fast and good wetting of the microparticles that may be injected without any issues using a 20 Gauge needle.

### Example 5: Release profile in vivo (rabbits)

Microparticles containing octreotide are suspended in 1 ml of a suitable aqueous vehicle and the resulting suspension is injected intramusculary (i.m.) into male New Zealand White rabbits in a dose of 12 mg/kg. For each dosage form (test group) 4 animals are used. After defined time periods (indicated in the table 4) plasma samples are taken and analyzed for octreotide concentration by radioimmunoassay (RIA).

**Table 4: Plasma levels Example 1-1**

| Time [days] / Subject No. | 473 | 474 | 476 | 480 | Mean or Range^{#} | SD |
|---|---|---|---|---|---|---|
| 0 | 0.000 | 0.000 | 0.000 | 0.000 | 0.000 | 0.000 |
| 0.021 | 56.026 | 41.316 | 52.099 | 48.148 | 49.397 | 6.274 |
| 0.042 | 40.769 | 50.921 | 37.531 | 30.494 | 39.929 | 8.491 |
| 0.083 | 16.154 | 25.658 | 15.185 | 11.889 | 17.222 | 5.913 |
| 0.167 | 4.590 | 5.408 | 4.654 | 2.617 | 4.317 | 1.19 |
| 0.25 | 2.103 | 1.987 | 1.383 | 1.006 | 1.620 | 0.517 |
| 1 | 0.763 | 0.597 | 0.503 | 0.517 | 0.595 | 0.119 |
| 2 | 0.579 | 0.694 | 0.513 | 0.476 | 0.566 | 0.096 |
| 6 | 1.769 | 2.105 | 1.556 | 1.802 | 1.808 | 0.226 |
| 9 | 2.218 | 2.89 | 2.099 | 1.864 | 2.269 | 0.442 |
| 16 | 2.744 | 2.750 | 2.198 | 2.136 | 2.457 | 0.336 |
| 23 | 2.436 | 3.111 | 2.185 | 2.049 | 2.447 | 0.475 |
| 30 | 2.192 | 2.579 | 1.741 | 2.173 | 2.171 | 0.342 |
| 37 | 2.564 | 3.526 | 2.049 | 2.605 | 2.686 | 0.614 |
| 44 | 1.731 | 3.053 | 1.667 | 2.420 | 2.218 | 0.653 |
| 51 | 2.589 | 2.355 | 1.259 | 2.914 | 2.279 | 0.718 |
| 58 | 2.128 | 1.842 | 1.104 | 2.975 | 2.012 | 0.773 |
| 65 | 1.206 | 1.684 | 0.712 | 2.333 | 1.484 | 0.691 |
| 72 | 0.631 | 1.056 | 0.613 | 1.358 | 0.915 | 0.360 |
| 79 | 0.21& | 0.600 | 0.389 | 0.837 | 0.511 | 0.268 |
| 86 | 0.111 | 0.219 | 0.143 | 0.425 | 0.225 | 0.141 |
| 93 | 0.000 | 0.105 | 0.000 | 0.231 | 0.084 | 0.110 |
| 100 | 0.000 | 0.000 | 0.000 | 0.111 | 0.028 | 0.056 |

## Claims

1. A depot pharmaceutical formulation in the form of microparticles comprising as active ingredient octreotide, or a pharmaceutically acceptable salt thereof, and two different linear polylactide-co-glycolide polymers (PLGAs) having a molar L:G ratio of 75:25 and wherein said two polymers have different inherent viscosities, wherein one polymer has an ester and the other polymer has an acid end-group, wherein said polymers have inherent viscosities between 0.1 dl/g and 0.8dl/g in CHCl₃ at 25 °C at a concentration of 0.1%, where the active ingredient which is used to prepare the depot formulation is in the form of an amorphous powder.

2. The depot pharmaceutical formulation of claim 1, wherein the amorphous powder of the active ingredient has a particle size of 0.1 microns to 15 microns (90 % > 0.1 microns, 99 % < 15 microns).

3. The depot pharmaceutical formulation of claim 1 or claim 2, wherein said polymers have inherent viscosities between 0.1 dl/g and 0.5dl/g in CHCl₃.

4. The depot pharmaceutical formulation according to any one of the claims 1 to 3 wherein the active ingredient is octreotide pamoate.

5. The depot pharmaceutical formulation composition according to claim 1 wherein the microparticles have a diameter between 10 µm and 90 µm.

6. The depot pharmaceutical formulation according to claim 1 or 5 wherein the microparticles are additionally covered or coated with an anti-agglomerating agent.

7. The depot pharmaceutical formulation according to any one of the claims 1 to 6 in the form of microparticles comprising octreotide pamoate and blend of 30% linear polymer PLGA 75:25 acid 0.2 dL/g (%) and 70% linear polymer PLGA 75:25 ester 0.4 dUg (%), with a drug load of 20 %, with a PLGA concentration of 20 % and a X₉₀ particle size of 60 micrometers.

8. The depot formulation according to any one of the claims 1 to 7 for use in the treatment of a disease selected from long-term maintenance therapy in acromegalic patients, severe diarrhea and flushing associated with malignant carcinoid tumors and vasoactive intestinal peptide tumors (vipoma tumors),

9. An administration kit comprising the depot formulation according to any one of the claims 1 to 7 in a vial, together with a water-based vehicle in an ampoule, vial or prefilled syringe or as microparticles and vehicle separated in a double chamber syringe.

## Patentansprüche

1. Pharmazeutische Depotformulierung in Form von Mikropartikeln, die als Wirkstoff Octreotid oder ein pharmazeutisch unbedenkliches Salz davon und zwei verschiedene geradkettige Polylactid-Co-Glycolid-Polymere (PLGAs) mit einem molaren L:G-Verhältnis von 75:25 umfassen, wobei die beiden Polymere unterschiedliche inhärente Viskositäten aufweisen, wobei ein Polymer eine Ester- und das andere Polymer eine Säure-Endgruppe aufweist, wobei die Polymere bei 25°C bei einer Konzentration von 0,1% in CHCl₃ inhärente Viskositäten zwischen 0,1 dl/g und 0,8 dl/g aufweisen, wobei der zur Herstellung der Depotformulierung verwendete Wirkstoff in Form eines amorphen Pulvers vorliegt.

2. Pharmazeutische Depotformulierung nach Anspruch 1, wobei das amorphe Pulver des Wirkstoffs eine Teilchengröße von 0,1 Mikron bis 15 Mikron (90% > 0,1 Mikron, 99% < 15 Mikron) aufweist.

3. Pharmazeutische Depotformulierung nach Anspruch 1 oder 2, wobei die Polymere in CHCl₃ inhärente Viskositäten zwischen 0,1 dl/g und 0,5 dl/g aufweisen.

4. Pharmazeutische Depotformulierung nach einem der Ansprüche 1 bis 3, wobei es sich bei dem Wirkstoff um Octreotidpamoat handelt.

5. Pharmazeutische Depotformulierungszusammensetzung nach Anspruch 1, wobei die Mikropartikel einen Durchmesser zwischen 10 µm und 90 µm aufweisen.

6. Pharmazeutische Depotformulierung nach Anspruch 1 oder 5, wobei die Mikropartikel zusätzlich mit einem Antiagglomerisierungsmittel beschichtet bzw. überzogen sind.

7. Pharmazeutische Depotformulierung nach einem der Ansprüche 1 bis 6 in Form von Mikropartikeln, die Octreotidpamoat und eine Mischung von 30% geradkettigem Polymer PLGA 75:25 Säure 0,2 dl/g (%) und 70% geradkettigem Polymer PLGA 75:25 Ester 0,4 dl/g (%) umfasst, mit einer Arzneimittelbeladung von 20%, mit einer PLGA-Konzentration von 20% und einer X₉₀-Teilchengröße von 60 Mikrometern.

8. Depotformulierung nach einem der Ansprüche 1 bis 7 zur Verwendung bei der Behandlung einer Krankheit ausgewählt aus langfristiger Erhaltungstherapie bei Patienten mit Akromegalie, schwerem Durchfall und Hitzewallungen assoziiert mit malignen karzinoiden Tumoren und vasoaktives intestinales Peptid sezernierenden Tumoren (Vipoma-Tumoren).

9. Verabreichungskit, umfassend die Depotformulierung nach einem der Ansprüche 1 bis 7 in einem Vial zusammen mit einem Vehikel auf Wasserbasis in einer Ampulle, einem Vial oder einer vorgefüllten Spritze oder als Mikropartikel und Vehikel getrennt in einer Spritze mit einer Doppelkammer.

## Revendications

1. Formulation pharmaceutique retard sous la forme de microparticules comprenant, en tant que substance active, l'octréotide, ou un sel pharmaceutiquement acceptable de celui-ci, et deux polymères polylactide-co-glycolide linéaires différents (PLGA) ayant un rapport molaire L:G de 75:25 et dans laquelle lesdits deux polymères ont des viscosités inhérentes différentes, dans laquelle un polymère comporte un ester et l'autre polymère comporte un groupe d'extrémité acide, dans laquelle lesdits polymères ont des viscosités inhérentes comprises entre 0,1 dl/g et 0,8 dl/g dans CHCl₃ à 25 °C à une concentration de 0,1 %, où la substance active qui est utilisée pour préparer la formulation retard est sous la forme d'une poudre amorphe.

2. Formulation pharmaceutique retard de la revendication 1, dans laquelle la poudre amorphe de la substance active a une taille de particule de 0,1 micron à 15 microns (90 % > 0,1 micron, 99 % < 15 microns).

3. Formulation pharmaceutique retard de la revendication 1 ou la revendication 2, dans laquelle lesdits polymères ont des viscosités inhérentes comprises entre 0,1 dl/g et 0,5 dl/g dans CHCl₃.

4. Formulation pharmaceutique retard selon l'une quelconque des revendications 1 à 3 dans laquelle la substance active est le pamoate d'octréotide.

5. Composition de formulation pharmaceutique retard selon la revendication 1 dans laquelle les microparticules ont un diamètre compris entre 10 µm et 90 µm.

6. Formulation pharmaceutique retard selon la revendication 1 ou 5 dans laquelle les microparticules sont en outre recouvertes ou revêtues avec un agent antiagglomérant.

7. Formulation pharmaceutique retard selon l'une quelconque des revendications 1 à 6 sous la forme de microparticules comprenant du pamoate d'octréotide et un mélange de 30 % de polymère linéaire PLGA 75:25 acide 0,2 dl/g (%) et 70 % de polymère linéaire PLGA 75:25 ester 0,4 dl/g (%), avec une charge de médicament de 20 %, avec une concentration de PLGA de 20 % et une taille de particule X₉₀ de 60 micromètres.

8. Formulation retard selon l'une quelconque des revendications 1 à 7 pour utilisation dans le traitement d'une maladie choisie parmi une thérapie d'entretien à long terme chez des patients acromégaliques, pour une diarrhée sévère et des bouffées vasomotrices associées à des tumeurs carcinoïdes malignes et des tumeurs sécrétrices de peptides intestinaux vasoactifs (tumeurs vipomes).

9. Trousse d'administration comprenant la formulation retard selon l'une quelconque des revendications 1 à 7 dans un flacon, conjointement avec un véhicule à base d'eau dans une ampoule, un flacon ou une seringue préremplie ou sous forme de microparticules et un véhicule séparé dans une seringue à chambre double.
